# EUROPEAN PATENT SPECIFICATION

(11) **EP 0 630 209 B1**
(45) Date of publication and mention of the grant of the patent: **23.09.1998**
(21) Application number: 93907483.7
(22) Date of filing: 12.03.1993
(51) Int. Cl.: A61B 6/12, A61B 6/00, A61B 17/34

(54) **ISOCENTRIC PUNCTURE INSTRUMENT AIMING DEVICE**
ZIELVORRICHTUNG FÜR EIN ISOZENTRISCHES PUNKTIONSINSTRUMENT
DISPOSITIF DE POINTAGE ISOCENTRIQUE DESTINE A UN INSTRUMENT DE PONCTION

(30) Priority: 12.03.1992 US 851683
(43) Date of publication of application: 28.12.1994
(73) Proprietor: FISCHER IMAGING CORPORATION, Denver, CO 80241 (US)
(72) Inventor: SICZEK, Bernard, W., Boulder, CO 80303-1411 (US); SICZEK, Aldona, A., Boulder, CO 80303-1411 (US)
(74) Representative: Pidgeon, Robert John
(86) International application number: PCT/US93/02308
(87) International publication number: WO 93/17620

(56) References cited:
- EP-A- 0 390 653
- EP-A- 0 483 005
- WO-A-90/05492
- FR-A- 861 311
- GB-A- 2 239 605
- US-A- 4 722 336
- US-A- 4 727 565
- US-A- 4 750 487
- US-A- 4 821 727
- US-A- 4 943 986
- US-A- 5 056 523
- US-A- 5 078 142
- US-A- 5 129 911

## Description

### FIELD OF THE INVENTION

The present invention relates to medical instruments used in diagnostic penetration of a body to collect a cell or tissue sample and, more particularly, to such an instrument incorporating an aiming device for aiming a puncture instrument to an isocenter of the instrument wherein a targeted object or area of interest is positioned. The present invention is particularly useful for sampling, excising or treating suspicious breast lesions.

### BACKGROUND OF THE INVENTION

The use of puncture instruments for collecting cell or tissue samples, e.g., from suspicious breast lesions, in diagnostic examinations is well known. Also well known is the use of imaging systems such as fluoroscopic imaging systems for locating an area of interest within a body and for aiming and guiding a needle. Since such systems ordinarily provide two dimensional images, two views are normally required for three-dimensional orientation.

Based on the two views, the spatial coordinates of an area of interest, e.g., a suspicious breast lesion, can be determined for use in aiming the puncture instrument. However, the penetration path of the puncture instrument thus defined is often obstructed by equipment or body parts, thereby necessitating selection of a different penetration path. Conventionally, selection of a different path entails re-imaging the area of interest and/or repeating calculations or alignment processes for aiming the puncture instrument. Such processes are time consuming, subject the patient to great stress and can result in errors due to fatigue or patient movement.

US 4943986 describes compression apparatus for a prosthetically augmented breast having an x-ray source with upper and lower compression plates mounted in one x-ray path.

### SUMMARY OF THE INVENTION

The present invention discloses an apparatus for aiming a puncture instrument to an area of interest within a patient's body and is particularly suitable for aiming a puncture instrument to an area of interest within the patient's breast. The present invention allows for real time, three-dimensional aiming of the puncture instrument to the area of interest, such as a suspicious breast lesion, by obtaining appropriate initial alignment of the area of interest in two imaging views. In addition, the present invention thereafter allows for real time selecting of the puncture instrument penetration path by pivoting the puncture instrument about the area of interest and observing the instrument on an imaging monitor or screen without the need for re-alignment, re-imaging or repeating calculations.

According to one aspect of the present invention, there is provided an apparatus for aiming a puncture instrument to an area of interest within a patient's breast, comprising:
apertured immobilization means for immobilizing said patient's breast relative to a predetermined frame of reference having a plurality of apertures, characterised by said apertures being located on opposing sides of said patient's breast to allow for selection from a plurality of differing directional orientations of a desired penetration path for accessing said area of interest using said puncture instrument;
imaging means disposed in predetermined relation to and movable relative to said predetermined frame of reference, for imaging said area of interest within said patient's breast;
isocentric support means disposed in predetermined relation to and movable relative to said predetermined frame of reference, for movably supporting said puncture instrument so that said instrument is movable across a range of positions relative to said predetermined flame of reference, wherein said puncture instrument is aimed at a common point, the isocenter in each position of said range of positions; and
positioning means for providing relative movement between said area of interest within said patient's breast and said isocenter of said isocentric support means so that said area of interest is located at said isocenter;
   wherein said isocentric support means allows for selective aiming of said puncture instrument at said area of interest positioned at said isocenter by way of any of various penetration paths having differing directional orientations through any of said plurality of apertures of said immobilization means located on opposing sides of said patient's breast.

Preferably, table means is provided for supporting said patient including an opening through which said patient's breast is permitted to pendulantly protrude into said predetermined frame of reference for immobilization of said patient's breast by said immobilization means.

Preferably, the apparatus includes arm means for supporting an imaging signal source and an imaging signal receiver for relative movement therebetween, wherein a distance between said imaging signal source and said imaging signal receiver can be changed by moving at least one of said imaging signal source and said imaging signal receiver.

Preferably, said immobilization means comprises:
first and second paddles disposed in an opposing relationship, each having said plurality of apertures therethrough and collectively defining a breast receiving space therebetween; and
biasing means for urging said first and second paddles together so as to compressingly engage said patient's breast.

Preferably, said immobilization means comprises a paddle formed to substantially match a contour of said patient's breast, said paddle having said plurality of apertures therethrough.

Preferably, said positioning means is operative for orbitally moving said puncture instrument across a range of positions relative to said isocenter, the range of positions defining a substantially planar region.

Preferably, said positioning means is operative for rotationally moving said puncture instrument across a range of orbital positions relative to said isocenter, the range of orbital positions defining a three dimensional region.

Preferably, said imaging means includes an imaging signal source and an imaging signal receiver, said imaging signal source and imaging signal receiver being supported in opposing relation by an arm means to define an imaging signal axis wherein there is a point on the imaging signal axis, the imaging isocenter, which point does not move in space when said imaging signal source and imaging signal receiver are rotatably moved in tandem via said arm means between a first imaging position and a second imaging position relative to said predetermined frame of interest. The imaging system includes an imaging signal source such as an x-ray imaging source, magnetic resonance imaging source or the like and an imaging signal receiver such as an x-ray film tray or a radiation sensitive device for substantially real time imaging. The imaging signal source and imaging signal receiver define an imaging signal axis which coincides with a center line of the radiation signal. The imaging system is constructed such that there is a point on the imaging signal axis, the isocenter, which does not move in space when the isocentric imaging means is moved between a first imaging position and a second imaging position. For example, the imaging system can be moved between the first and second imaging positions by rotating the imaging signal source and imaging signal receiver about an axis extending through the isocenter. Preferably, the imaging system is isocentric with respect to at least one such rotational axis.

According to another aspect of the present invention there is provided an apparatus for aiming a puncture instrument to an area of interest within a patient's breast, characterised by comprising:
isocentric imaging means for imaging said area of interest within said patient's breast including an imaging signal source and an imaging signal receiver, the imaging signal source and imaging signal receiver being supported in opposing relation by an arm means to define an imaging signal axis wherein there is a point on the imaging signal axis, the imaging isocenter, which point does not move in space when the isocentric imaging signal source and imaging signal receiver are rotatably moved in tandem via said arms means between a first imaging position and a second imaging position;
isocentric support means interconnected to said isocentric imaging means, for movably supporting said puncture instrument so that said puncture instrument is movable across a range of aiming positions including a first aiming position and a second aiming position, wherein said puncture instrument is aimed at said isocenter in each of said first and second aiming positions, wherein said isocentric imaging means and said isocentric support means share a common isocenter; and
positioning means for providing relative movement between said area of interest within said patient's breast and said isocenter so that said area of interest within said patient's breast is located at said isocenter.

Preferably, said arm means supports said imaging signal source, imaging signal receiver and said support means, and said positioning means comprises means for moving the arm means.

Preferably, table means is provided for supporting said patient, wherein said arm means supports said imaging signal source and said imaging signal receiver so that said imaging signal source and said imaging signal receiver are disposed on the same side of said table means.

Preferably, the apparatus includes table means for supporting said patient including an opening through which said patient's breast is permitted to pendulantly protrude, wherein said arm means supports said imaging signal source and said imaging signal receiver relative to said pendulant breast so as to permit imaging of said pendulant breast.

Preferably, said arm means supports said imaging signal source and said imaging signal receiver for relative movement therebetween, wherein a distance between said imaging signal source and said imaging signal receiver can be changed by moving at least one of said imaging signal source and said imaging signal receiver.

Preferably, said apparatus further comprises immobilization means for immobilizing said patient's breast.

Preferably, said isocentric imaging system comprises a monitor associated with said imaging signal receiver to provide substantially real time imaging. The apparatus for aiming a puncture instrument supports a puncture instrument so that the puncture instrument is movable between at least a first position and a second position wherein the puncture instrument is aimed at the isocenter in each of the first and second positions. It will thus be appreciated that the puncture instrument, like the imaging system, is isocentric and that the puncture instrument and imaging system share a common isocenter, i.e., there is a point on an aiming axis of the puncture instrument, the isocenter, which does not move in space when the puncture instrument is moved between the first and second positions. In this regard, for example, the puncture instrument can be orbitally moved relative to the isocenter so that the puncture instrument pivots thereabout within a single plane (defined by movement of the aiming axis) and/or the puncture instrument can be rotationally moved relative to the isocenter so that the puncture instrument pivots thereabout in three-dimensions.

In one embodiment, the apparatus for aiming a puncture instrument comprises a table having an opening therein through which a patient's breast is permitted to pendulantly protrude. The imaging signal source, imaging signal receiver and puncture instrument are disposed beneath the table and are movable around the patient's breast. At least one compression paddle is provided to immobilize the breast during the procedure. Preferably, a pair of paddles formed to substantially match a contour of the patient's breast are provided. A biasing assembly, such as a spring or pneumatic device, is employed to urge the paddles together such that the patient's breast is compressingly engaged therebetween. At least one of the paddles has openings, of any shape, to permit passage of the puncture instrument.

In operation, an initial calibration procedure is performed without the patient to ensure that the puncture instrument is aimed at the isocenter. This can be accomplished by simply viewing the alignment of the instrument tip with the instrument in a number of different positions. It will be appreciated that, when the tip is located at the isocenter, the puncture instrument can be moved to different positions without movement of the tip. The puncture instrument is then withdrawn or removed to allow the patient to be situated on the table. The table and/or the isocenter are then moved until the area of interest is located at the isocenter. An operator knows that the area of interest is located at the isocenter when the area of interest does not move when the imaging equipment is moved from one position to another. Three-dimensional, real time aiming can thus be achieved if real time imaging equipment is employed. Additionally, a penetration path to an area of interest within the patient's breast via an opening in a paddle can be rapidly selected by simply pivoting the puncture instrument. It will thus be appreciated that the apparatus is well suited for use in biopsy procedures to obtain a cell or tissue sample from a suspicious breast lesion.

In another embodiment, the apparatus for aiming a puncture instrument includes a table for supporting the patient. An x-ray tube and an x-ray image receptor (for example, an x-ray film tray or a charge-coupled device for providing substantially real-time imaging via a monitor) are disposed at opposite ends of a "C" shaped or "U" shaped arm, or are otherwise disposed in a movable, opposing relationship for imaging an area of interest within the patient's body. One of the x-ray tube and x-ray image receptor is positioned above the table and the other is positioned below. The x-ray tube and x-ray image receptor are rotatable or otherwise movable such that the isocenter located on the imaging signal axis remains fixed, i.e., the radiation axis is rotatable about the isocenter.

The puncture instrument is mounted on the apparatus such that the instrument is pivotally movable about the isocenter. That is, the instrument remains pointed at the isocenter as the instrument is moved from one position to another. Conveniently, the puncture instrument, x-ray tube and x-ray image receptor can be mounted on a common arm which is rotatable about the isocenter. Additionally, the puncture instrument is mounted such that it is rotatable about a second axis, which extends through the isocenter, and is movable on an arcuate rail assembly or the like for further pivotal movement about the isocenter. The puncture instrument can be a biopsy needle, a cannula for providing access to the area of interest for other medical instruments, a needle for delivering a medicament or other instruments depending on the particular application. The puncture instrument can be retained in a guide for manual insertion or in an automatic delivery system such as a biopsy gun. The apparatus operates in a manner substantially identical to that described above. The puncture instrument penetration path can be changed, for example, to avoid vital organs or ribs, by simply pivoting the puncture instrument.

### BRIEF DESCRIPTION OF THE DRAWINGS

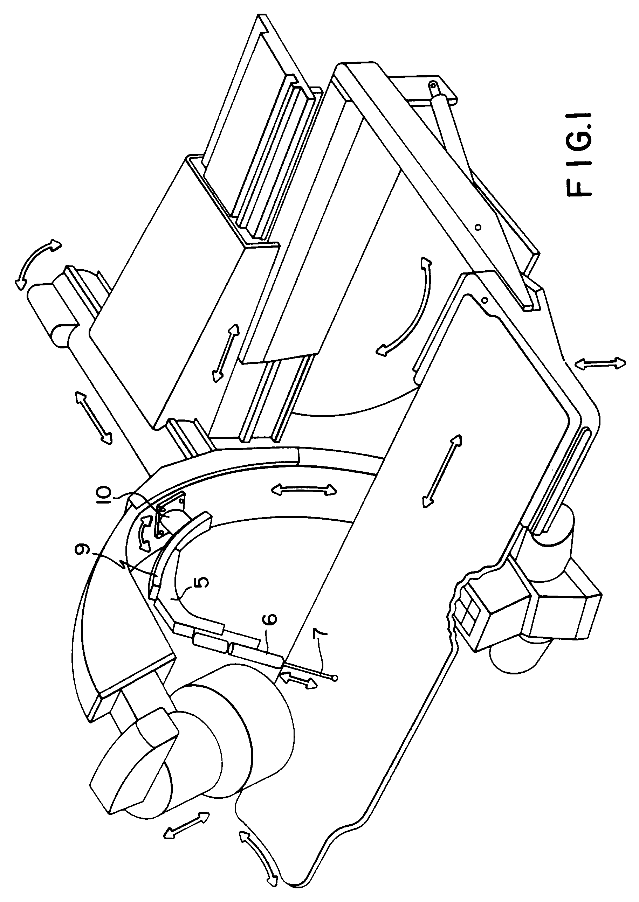

Fig. 1 is a pictorial view of an apparatus constructed in accordance with the present invention including C-arm fluoroscopic equipment;
Fig. 2 is a pictorial view of a further apparatus constructed in accordance with the present invention including U-arm fluoroscopic equipment;
Fig. 3 is an elevation view of the apparatus of Fig. 1;
Fig. 4 is a side view of a still further apparatus constructed in accordance with the present invention; and
Fig. 5 is a bottom view of the apparatus of Fig. 4 showing mammo compression paddles compressing a patient's breast, a targeted object overlying an isocenter of the radiation axis and a puncturing needle aiming at the targeted object.

### DETAILED DESCRIPTION

The present invention discloses an isocentric puncture instrument aiming device. The device includes an imaging system and a movable puncture instrument which share a common isocenter. The present invention thus takes advantage of built-in isocentric device geometry to provide for real-time aiming and selection of a puncture instrument penetration path without the need for realignment, re-imaging of the patient or repeating calculations. The invention will first be described with respect to embodiments generally applicable for use in connection with various body regions. Thereafter, the invention will be described with respect to embodiments which are particularly adaptable for use in connection with the breast.

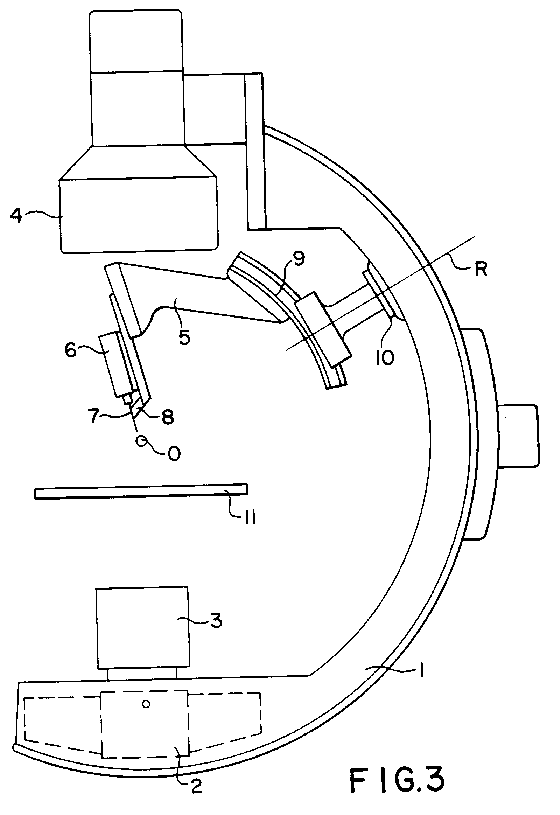

Referring to Fig. 3, the orbital aiming device comprises a C-arm 1 carrying an x-ray tube 2 with a collimator 3 having cross-hairs in the center of the beam and an image receptor 4. The C-arm is isocentric about two rotational axes with the isocenter at point O on the radiation axis defined by the x-ray tube and the image receptor. The isocenter remains at the same point O in space when the radiation axis is rotated about the two rotational axes.

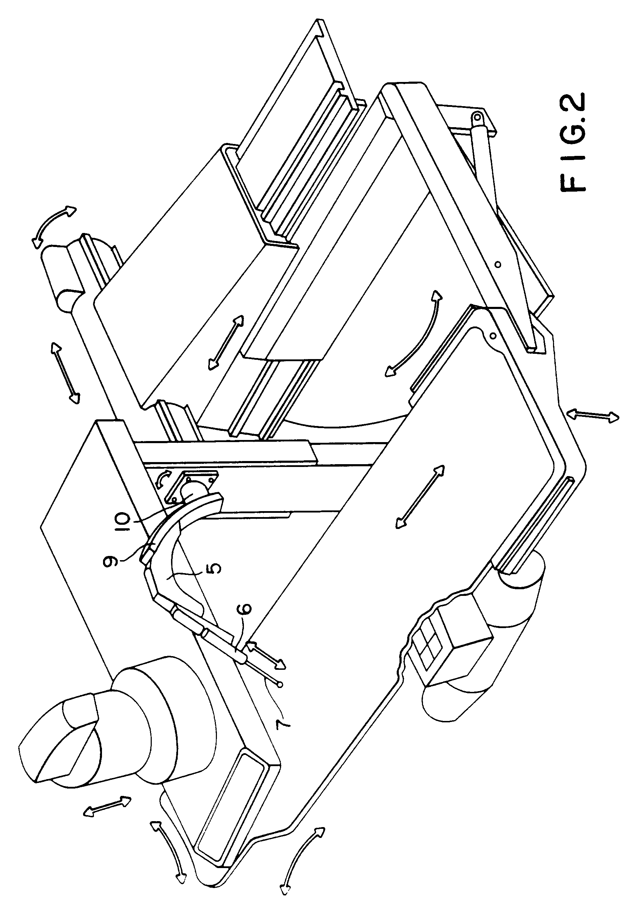

The orbital aiming device further comprises an elongated member 5 having a biopsy gun 6 with a needle 7 movably mounted thereon so that the needle aims at the isocenter O; the biopsy gun with the needle is linearly displaceable to a manually adjusted stop 8 which controls the depth of the needle penetration. The elongated member 5 is mounted on an arcuate rail system 9 for a relative orbital movement therebetween and, hence, for pivotal movement of the needle tip about the isocenter O. The arcuate rail system 9 is shown to include two arcuate components coupled in a telescopic relationship for providing an extended orbital travel of the elongated member 5 without compromising compactness of the device. The arcuate rail system 9 is rotatably attached to a rotational means 10 and rotatable about an axis R for pivotal movement of the needle tip about the isocenter. The rotational means is attached, for example, to the C-arm or U-arm (Fig. 2) by a connecting means providing for prompt attachment and detachment of the device if desirable to gain more access to a patient. The connecting means provides for attachment such that the isocenter of the rotational puncture device overlies the isocenter of the fluoroscopic imaging system.

An initial calibration of the orbital aiming device is carried out without a patient by viewing the needle on the fluoroscopic screen to align it to the cross-hair in one view and to adjust the stop 8 in the second view so that the needle points to the cross-hairs.

Then, the patient is positioned on a movable table top 11 and the table top is moved in three directions so that the targeted object overlies the isocenter, that is, overlies the cross-hairs in at least two views. The needle tip is now pivoted for selecting a path of penetration, so that vital organs are avoided and the needle aims between ribs.

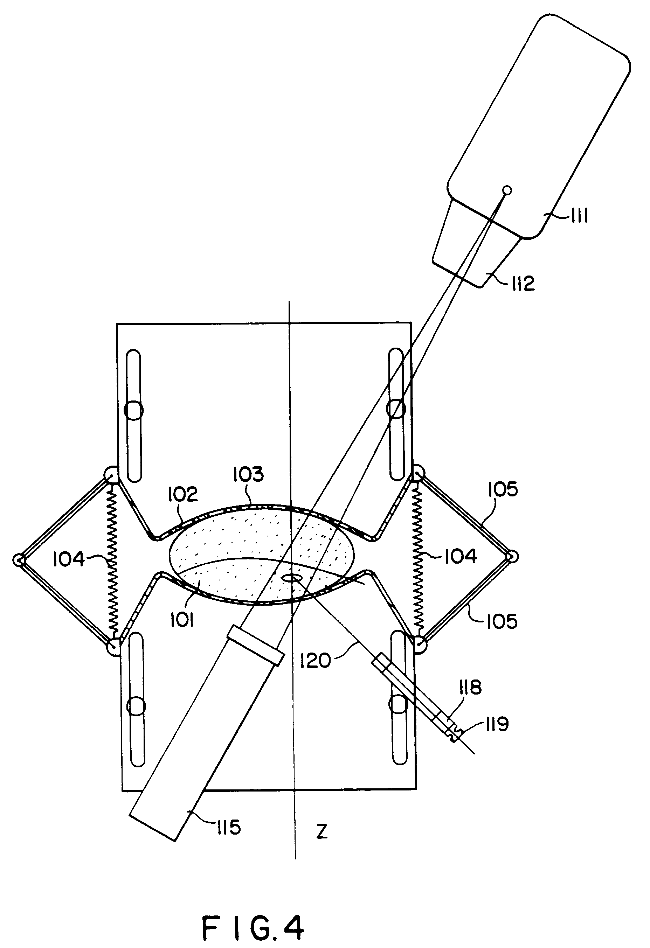

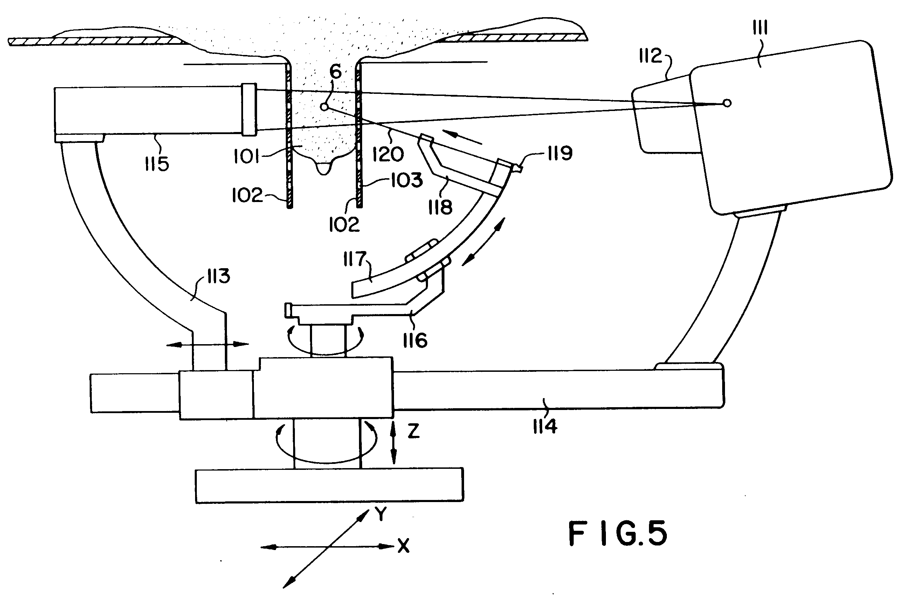

Referring to Figs. 4-5, the orbital aiming device for breast biopsy comprises mammo fluoro x-ray equipment including an x-ray tube 111 with a collimator 112 and an image receptor 115, which x-ray tube and image receptor define the radiation axis.

Cross-hairs indicating the center of the beam are placed either on the collimator or the image receptor to assist in the alignment.

A patient breast 101 is compressed between lens shaped compression paddles 102 translucent to the x-rays, which have holes 103 for inserting the biopsy needle 120.

The compression paddles are rotatable around the breast and displaceable relative to the breast, away from the breast by means of arms 105 and toward compressing the breast by action of springs 104.

An elongated arm 114 supporting diametrically the x-ray tube at one end and a fluoro image receptor at the other by means of the arm 113 slidably mounted thereon, is linearly displaceable along the x,y,z orthogonal coordinates and rotatable around the z-coordinate, wherein the z-coordinate passes through the isocenter.

The fluoro image receptor can be a tubular image intensifier or a fiber optics reducer with a TV camera having a vacuum tube or being solid state.

The orbital aiming device comprises a guide member 118 having a puncture instrument 120 movably mounted thereon so that the needle aims at the isocenter; the needle is linearly displaceable to a manually adjusted stop 119 which controls the depth of its penetration. The guide member 118 is affixed to an arcuate member 117, which member is mounted on a swivel arm 116 and adapted for a relative orbital movement therebetween and, hence, for pivotal movement of the needle tip about the isocenter. The swivel arm 116 is rotatably mounted on the elongated arm 114 and rotatable about the z axis for pivotal movement of the needle tip about the isocenter.

Again, an initial calibration of the orbital aiming device is carried out by viewing the alignment of the tip of the needle to the cross-hairs in a number of angulated positions of the needle and of the image receptor; the stop 119 is adjusted at that time.

To overlie the isocenter and the targeted object, the targeted object is aligned to the cross-hairs in at least two views by rotating and linearly displacing the arm 114. By swiveling the arm 116 and orbitally displacing the arcuate member 117, a penetration path passing through either of the compression paddles is selected.

## Claims

1. An apparatus for aiming a puncture instrument (7, 120) to an area of interest within a patient's breast (101), comprising:
apertured immobilization means (102) for immobilizing said patient's breast relative to a predetermined frame of reference having a plurality of apertures (103), characterised by said apertures being located on opposing sides of said patient's breast to allow for selection from a plurality of differing directional orientations of a desired penetration path for accessing said area of interest using said puncture instrument (7, 120);
imaging means (2,4; 111, 115), disposed in predetermined relation to and movable relative to said predetermined frame of reference, for imaging said area of interest within said patient's breast;
isocentric support means (5, 6, 9, 10; 116, 117, 118), disposed in predetermined relation to and movable relative to said predetermined frame of reference, for movably supporting said puncture instrument so that said instrument is movable across a range of positions relative to said predetermined frame of reference, wherein said puncture instrument is aimed at a common point, the isocenter (0), in each position of said range of positions; and
positioning means for providing relative movement between said area of interest within said patient's breast and said isocenter of said isocentric support means so that said area of interest is located at said isocenter;
wherein said isocentric support means allows for selective aiming of said puncture instrument at said area of interest positioned at said isocenter by way of any of various penetration paths having differing directional orientations through any of said plurality of apertures (103) of said immobilization means (102) located on opposing sides of said patient's breast.

2. Apparatus according to claim 1, further comprising:
table means (11) for supporting said patient including an opening through which said patient's breast is permitted to pendulantly protrude into said predetermined frame of reference for immobilization of said patient's breast by said immobilization means.

3. Apparatus according to any preceding claim, further comprising:
arm means (1, 14) for supporting an imaging signal source (2, 111) and an imaging signal receiver (4, 115) for relative movement therebetween, wherein a distance between said imaging signal source and said imaging signal receiver can be changed by moving at least one of said imaging signal source and said imaging signal receiver.

4. Apparatus according to any preceding claim, wherein said immobilization means comprises:
first and second paddles (102) disposed in an opposing relationship, each having said plurality of apertures (103) therethrough and collectively defining a breast (101) receiving space therebetween; and
biasing means (104) for urging said first and second paddles together so as to compressingly engage said patient's breast.

5. Apparatus according to any of claims 1 to 3, wherein said immobilization means comprises a paddle (102) formed to substantially match a contour of said patient's breast, said paddle having said plurality of apertures (103) therethrough.

6. Apparatus according to any preceding claim, wherein said positioning means (9, 117) is operative for orbitally moving said puncture instrument across a range of positions relative to said isocenter, the range of positions defining a substantially planar region.

7. Apparatus according to any preceding claim, wherein said positioning means (10, 116) is operative for rotationally moving said puncture instrument across a range of orbital positions relative to said isocenter, the range of orbital positions defining a three dimensional region.

8. Apparatus according to any preceding claim, wherein said imaging means includes an imaging signal source (2, 111) and an imaging signal receiver (4, 115), said imaging signal source and imaging signal receiver being supported in opposing relation by an arm means (1, 114) to define an imaging signal axis wherein there is a point on the imaging signal axis, the imaging isocenter, which point does not move in space when said imaging signal source and imaging signal receiver are rotatably moved in tandem via said arm means between a first imaging position and a second imaging position relative to said predetermined frame of interest.

9. An apparatus for aiming a puncture instrument (7, 120) to an area of interest within a patient's breast (101), characterised by comprising:
isocentric imaging means (2, 4; 111, 115) for imaging said area of interest within said patient's breast including an imaging signal source (2, 111) and an imaging signal receiver (4, 115), the imaging signal source and imaging signal receiver being supported in opposing relation by an arm means (1, 114) to define an imaging signal axis wherein there is a point on the imaging signal axis, the imaging isocenter, which point does not move in space when the isocentric imaging signal source and imaging signal receiver are rotatably moved in tandem via said arm means between a first imaging position and a second imaging position;
isocentric support means (5, 6, 9, 10; 116, 117, 118), interconnected to said isocentric imaging means, for movably supporting said puncture instrument so that said puncture instrument is movable across a range of aiming positions including a first aiming position and a second aiming position, wherein said puncture instrument is aimed at said isocenter in each of said first and second aiming positions, wherein said isocentric imaging means and said isocentric support means share a common isocenter; and
positioning means for providing relative movement between said area of interest within said patient's breast and said isocenter so that said area of interest within said patient's breast is located at said isocenter.

10. Apparatus according to claim 9, wherein said arm means supports said imaging signal source, imaging signal receiver and said support means, and said positioning means comprises means for moving the arm means.

11. Apparatus according to claim 9 or any succeeding claim, further comprising:
table means (11) for supporting said patient, wherein said arm means supports said imaging signal source and said imaging signal receiver so that said imaging signal source and said imaging signal receiver are disposed on the same side of said table means.

12. Apparatus according to claim 9 or any succeeding claim, further comprising:
table means (11) for supporting said patient including an opening through which said patient's breast is permitted to pendulantly protrude, wherein said arm means supports said imaging signal source and said imaging signal receiver relative to said pendulant breast so as to permit imaging of said pendulant breast.

13. Apparatus according to claim 9 or any succeeding claim, wherein said arm means supports said imaging signal source and said imaging signal receiver for relative movement therebetween, wherein a distance between said imaging signal source and said imaging signal receiver can be changed by moving (113) at least one of said imaging signal source and said imaging signal receiver.

14. Apparatus according to claim 9 or any succeeding claim, wherein said apparatus further comprises immobilization means (102) for immobilizing said patient's breast.

15. Apparatus according to claim 9 or any succeeding claim, wherein said isocentric imaging system comprises a monitor associated with said imaging signal receiver to provide substantially real time imaging.

## Patentansprüche

1. Apparatur zur Ausrichtung eines Punktionsinstruments (7, 120) auf einen interessierenden Bereich innerhalb einer Brust eines Patienten (101), der umfaßt:
löchrige Feststelleinrichtungen (102) zum Feststellen der Patientenbrust in bezug auf einen vorbestimmten Referenzrahmen, die eine Vielzahl von Öffnungen (103) aufweisen und dadurch charakterisiert sind, daß die Öffnungen an gegenüberliegenden Seiten der Patientenbrust angebracht sind, um eine Auswahl aus einer Vielzahl von verschiedenen Ausrichtungen eines gewünschten Eindringpfades zum Zugang zum interessierenden Bereich mittels des Punktionsinstruments (7, 120) zu ermöglichen;
Abbildungseinrichtungen (2, 4; 111, 115), die in in einer vorbestimmten Beziehung zu und beweglich in bezug auf den vorbestimmten Referenzrahmen angebracht sind, zur Abbildung des interessierenden Bereichs innerhalb der Patientenbrust;
isozentrische Halteeinrichtungen (5, 6, 9, 10; 116, 117, 118), die in vorbestimmter Beziehung zu und beweglich gegenüber dem vorbestimmten Referenzrahmen angebracht sind, zum beweglichen Halten des Punktionsinstruments, so daß das Instrument über einen Bereich in bezug auf den vorbestimmten Referenzrahmen beweglich ist, wobei das Punktionsinstrument auf einen gemeinsamen Punkt, das Isozentrum (0) in jeder Position des genannten Positionsbereichs gerichtet ist; und
Positioniereinrichtungen zur Erzeugung einer relativen Bewegung zwischen dem besagten interessierenden Bereich innerhalb der Patientenbrust und dem Isozentrum der isozentrischen Halteeinrichtungen, so daß der interessierende Bereich in dem Isozentrum positioniert ist;
**dadurch gekennzeichnet, daß** die isozentrischen Halteeinrichtungen eine selektive Ausrichtung des Punktionsinstruments auf den interessierenden Bereich, der an dem Isozenter positioniert ist, durch einen der verschiedenen Eindringpfade ermöglicht, die unterschiedliche Ausrichtungen durch eine aus der Vielzahl der Öffnungen (103) der Feststelleinrichtungen (102), die auf gegenüberliegenden Seiten der Patientenbrust angebracht sind, aufweisen.

2. Apparatur nach Anspruch 1, die weiterhin umfaßt:
Auflageeinrichtung (11)zur Aufnahme des Patienten, die eine Öffnung umfaßt, durch welche ermöglicht wird, daß die Patientenbrust hängend in den Referenzrahmen hineinragt zur Feststellung der Patientenbrust durch die Feststelleinrichtungen.

3. Apparatur nach einem der vorhergehenden Ansprüche, die weiterhin umfaßt:
Tragarme (1, 14) zum Tragen eines Abbildungssignalgebers (2, 111) und eines Abbildungssignalempfängers (4, 115) und zur relativen Bewegung zwischen diesen, wobei eine Entfernung zwischen dem Abbildungssignalgeber und dem Abbildungssignalempfänger durch die Bewegung von zumindest einem von dem Abbildungssignalgeber und dem Abbildungssignalempfänger verändert werden kann.

4. Apparatur nach einem der vorhergehenden Ansprüche, **dadurch gekennzeichnet, daß** die Feststelleinrichtungen umfassen:
eine erste und zweite Führung (102), die gegenüberliegend angebracht sind, wobei jede eine Vielzahl von Öffnungen (103) aufweist, durch die gemeinsam ein dazwischenliegender, die Brust aufnehmender Raum definiert wird; und Vorspanneinrichtungen (104) zum Zusammendrücken der ersten und zweiten Führung, so daß die Patientenbrust zusammendrückend umfaßt wird.

5. Apparatur nach einem der Ansprüche 1 bis 3, **dadurch gekennzeichnet, daß** die Feststelleinrichtungen eine Führung (102) umfassen, die so geformt ist, daß sie im wesentlichen der Form einer Patientenbrust entspricht, und die eine Vielzahl von Öffnungen (103) aufweist.

6. Apparatur nach einem der vorhergehenden Ansprüche, **dadurch gekennzeichnet, daß** die Positioniereinrichtungen (9, 117) betrieben werden, um das Punktionsinstrument über einen Positionsbereich relativ zu dem Isozentrum orbital zu bewegen, wobei der Positionsbereich einen im wesentlichen planaren Bereich definiert.

7. Apparatur nach einem der vorhergehenden Ansprüche, **dadurch gekennzeichnet, daß** die Positioniereinrichtungen (10, 116) betrieben werden, um eine Rotationsbewegung des Punktionsinstruments über einen Bereich von Orbitalpositionen relativ zu dem Isozentrum auszuführen, wobei der Bereich der Orbitalpositionen einen dreidimensionalen Bereich definiert.

8. Apparatur nach einem der vorhergehenden Ansprüche, **dadurch gekennzeichnet, daß** die Abbildungseinrichtungen einen Abbildungssignalgeber (2, 111) und einen Abbildungssignalempfänger (4, 115) umfassen, wobei der Abbildungssignalgeber und der Abbildungssignalempfänger gegenüberliegenderweise durch die Tragarmeinrichtungen (1, 114) gehalten werden, um eine Abbildungssignalachse zu definieren, in welcher sich ein Punkt befindet, das Abbildungsisozentrum, der sich im Raum nicht bewegt, wenn der Abbildungssignalgeber und der Abbildungssignalempfänger im Tandem über die Tragarmeinrichtungen zwischen einer ersten Abbildungsposition und einer zweiten Abbildungsposition relativ zu dem vorbestimmten Interessensbereich drehend bewegt werden.

9. Apparatur zur Ausrichtung eines Punktionsinstruments (7, 120) auf einen interessierenden Bereich innerhalb einer Patientenbrust (101), **dadurch gekennzeichnet, daß** er umfaßt:
isozentrische Abbildungseinrichtungen (2, 4; 111, 115) zur Abbildung des interessierenden Bereichs innerhalb der Patientenbrust, die ein Abbildungssignalgeber (2, 111) und ein Abbildungssignalempfänger (4, 115) umfassen, wobei der Abbildungssignalgeber und der Abbildungssignalempfänger in gegenüberliegender Weise durch Tragarmeinrichtungen (1, 114) gehalten werden, um eine Abbildungssignalachse zu definieren, in welcher ein Punkt existiert, das Abbildungsisozentrum, der sich nicht im Baum bewegt, wenn der isozentrische Abbildungssignalgeber und Abbildungssignalempfänger im Tandem über die Tragarmeinrichtungen zwischen einer ersten Abbildungsposition und einer zweiten Abbildungsposition drehend bewegt werden;
isozentrische Halteeinrichtungen (5, 6, 9, 10; 116, 117, 118), die mit den isozentrischen Abbildungseinrichtungen verbunden sind, zum beweglichen Halten des Punktionsinstruments, so daß das Punktionsinstrument über einen Bereich von Zielpositionen, einschließlich einer ersten Zielposition und einer zweiten Zielposition beweglich ist, wobei das Punktionsinstrument in jeder der ersten und zweiten Zielposition auf das genannte Isozentrum gerichtet ist und wobei die isozentrischen Abbildungseinrichtungen und die isozentrischen Halteeinrichtungen ein gemeinsames Isozentrum aufweisen; und
Positioniereinrichtungen zur Ermöglichung einer relativen Bewegung zwischen dem interessierenden Bereich innerhalb der Patientenbrust und dem Isozentrum, so daß der interessierende Bereich innerhalb der Patientenbrust an dem Isozentrum positioniert wird.

10. Apparatur nach Anspruch 9, **dadurch gekennzeichnet, daß** die Tragarmeinrichtungen den Abbildungssignalgeber, den Abbildungssignalempfänger und die Halteeinrichtungen tragen, und daß die Positioniereinrichtungen Einrichtungen zum Bewegen der Tragarmeinrichtungen umfassen.

11. Apparatur nach Anspruch 9 oder einem nachfolgenden Anspruch, der weiterhin umfaßt:
Liegetisch (11) zur Aufnahme des Patienten, wobei die Tragarmeinrichtungen den Abbildungssignalgeber und den Abbildungssignalempfänger halten, so daß der Abbildungssignalgeber und der Abbildungssignalempfänger auf der gleichen Seite des Liegetisches angebracht sind.

12. Apparatur nach Anspruch 9 oder einem nachfolgenden Anspruch, die weiterhin umfaßt:
Liegetisch (11) zur Aufnahme des Patienten, der eine Öffnung umfaßt, durch welche es ermöglicht wird, daß eine Patientenbrust hängend hindurchragt, wobei die Tragarmeinrichtungen den Abbildungssignalgeber und den Abbildungssignalempfänger relativ zu der hängenden Brust so trägt, daß sie die Abbildung der hängenden Brust ermöglichen.

13. Apparatur nach Anspruch 9 oder einem der nachfolgenden Ansprüche, **dadurch gekennzeichnet, daß** die Tragarmeinrichtungen den Abbildungssignalgeber und den Abbildungssignalempfänger zur relativen Bewegung zwischen diesen tragen, wobei eine Entfernung zwischen dem Abbildungssignalgeber und dem Abbildungssignalempfänger durch Bewegung (113) von mindestens einem der Abbildungssignalgeber oder Abbildungssignalempfänger verändert werden kann.

14. Apparatur nach Anspruch 9 oder einem der nachfolgenden Ansprüche, **dadurch gekennzeichnet, daß** die Apparatur weiterhin Feststelleinrichtungen (102) zum Feststellen der Patientenbrust umfaßt.

15. Apparatur nach Anspruch 9 oder einem der nachfolgenden Ansprüche, **dadurch gekennzeichnet, daß** das isozentrische Abbildungssystem einen Monitor umfaßt, der mit dem Abbildungssignalempfänger verbunden ist, um im wesentlichen Echtzeitabbildungen zu liefern.

## Revendications

1. Appareil pour pointer un instrument de ponction (7, 120) vers une zone d'intérêt dans le sein (101) d'un patient, comprenant :
un moyen d'immobilisation (102) muni d'ouvertures pour immobiliser ledit sein du patient par rapport à un cadre de référence prédéterminé ayant une pluralité d'ouvertures (103), caractérisé en ce que lesdites ouvertures sont situées sur des côtés opposés dudit sein du patient pour permettre un choix parmi une pluralité d'orientations directionnelles différentes d'un trajet de pénétration voulu pour accéder à ladite zone d'intérêt au moyen dudit instrument de ponction (7, 120);
des moyens d'imagerie (2, 4 ; 111, 115), disposés dans une relation prédéterminée avec et mobiles par rapport audit cadre de référence prédéterminé, pour l'imagerie de ladite zone d'intérêt dans ledit sein du patient;
des moyens de support isocentriques (5, 6, 9, 10 ; 116, 117, 118), disposés dans une relation prédeterminée avec et mobiles par rapport audit cadre de référence prédéterminé, pour supporter de manière mobile ledit instrument de ponction de sorte que ledit instrument est mobile sur une plage de positions par rapport audit cadre de référence prédéterminé, où ledit instrument de ponction est pointé vers un point commun, l'isocentre (0), dans chaque position de ladite plage de positions ; et
des moyens de positionnement pour lettre un mouvement relatif entre ladite zone d'intérêt dans ledit sein du patient et ledit isocentre desdits moyens de support isocentriques de sorte que ladite zone d'intérêt est située audit isocentre ;
où lesdits moyens de support isocentriques permettent le pointage sélectif dudit instrument de ponction au niveau de ladite zone d'intérêt positionnée audit isocentre au moyen d'un trajet de pénétration quelconque parmi différents trajets de pénétration ayant des orientations directionnelles différentes au travers de l'une quelconque de ladite pluralité d'ouvertures (103) dudit moyen d'immobilisation (102) situées sur des côtés opposés dudit sein du patient.

2. Appareil selon la revendication 1, comprenant en outre :
un moyen formant table (11) pour supporter ledit patient comprenant une ouverture par laquelle ledit sein du patient est amené à faire saillie de manière pendante dans ledit cadre de référence prédéterminé pour l'immobilisation dudit sein du patient par ledit moyen d'immobilisation.

3. Appareil selon l'une quelconque des revendications précédentes, comprenant en outre :
un moyen formant bras (1, 14) pour supporter une source de signaux d'imagerie (2, 111) et un récepteur de signaux d'imagerie (4, 115) pour un mouvement relatif entre eux, où une distance entre ladite source de signaux d'imagerie et ledit récepteur de signaux d'imagerie peut être modifiée en déplaçant au moins un élément parmi ladite source de signaux d'imagerie et ledit récepteur de signaux d'imagerie.

4. Appareil selon l'une quelconque des revendications précédentes, où ledit moyen d'immobilisation comprend :
des première et seconde palettes (102) disposées dans une relation d'opposition, traversées chacune par ladite pluralité d'ouvertures (103) et définissant collectivement entre elles un espace de réception de sein (101) ; et
des moyens de sollicitation (104) pour presser ensemble lesdites première et seconde palettes de manière à venir en contact par compression avec ledit sein du patient.

5. Appareil selon l'une quelconque des revendications 1 à 3, où ledit moyen d'immobilisation comprend une palette (102) formée de manière à coïncider sensiblement avec un contour dudit sein du patient, ladite palette état traversée par ladite pluralité d'ouvertures (103).

6. Appareil selon l'une quelconque des revendications précédentes, où lesdits moyens de positionnement (9, 117) sont actifs pour déplacer de manière orbitale ledit instrument de ponction sur une plage de positions par rapport audit isocentre, la plage de positions définissant une région sensiblement plane.

7. Appareil selon l'une quelconque des revendications précédentes, où lesdits moyens de positionnement (10, 116) sont actifs pour déplacer par rotation ledit instrument de ponction sur une plage de positions orbitales par rapport audit isocentre, la plage de positions orbitales définissant une région tridimensionnelle.

8. Appareil selon l'une quelconque des revendications précédentes, où lesdits moyens d'imagerie comprennent une source de signaux d'imagerie (2, 111) et un récepteur de signaux d'imagerie (4, 115), ladite source de signaux d'imagerie et ledit récepteur de signaux d'imagerie étant supportés dans une relation d'opposition par un moyen formant bras (1, 114) pour définir un axe de signaux d'imagerie où il existe un point sur l'axe de signaux d'imagerie, l'isocentre d'imagerie, lequel point ne se déplace pas dans l'espace lorsque ladite source de signaux d'imagerie et ledit récepteur de signaux d'imagerie sont déplacés en rotation en tandem par l'intermédiaire dudit moyen forment bras entre une première position d'imagerie et une seconde position d'imagerie par rapport audit cadre d'intérêt prédéterminé.

9. Appareil pour pointer un instrument de ponction (7, 120) vers une zone d'intérêt dans un sein (101) d'un patient, caractérisé en ce qu'il comprend :
des moyens d'imagerie isocentriques (2, 4 ; 111, 115) pour l'imagerie de ladite zone d'intérêt dans ledit soin du patient comprenant une source de signaux d'imagerie (2, 111) et un récepteur de signaux d'imagerie (4, 115), ladite source de signaux d'imagerie et ledit récepteur de signaux d'imagerie étant supportés dans une relation d'opposition par un moyen formant bras (1, 114) pour définir un axe de signaux d'imagerie où il existe un point sur l'axe de signaux d'imagerie, l'isocentre d'imagerie, lequel point ne se déplace pas dans l'espace lorsque la source de signaux d'imagerie et le récepteur de signaux d'imagerie isocentriques sont déplacés en rotation en tandem par l'intermédiaire dudit moyen formant bras entre une première position d'imagerie et une seconde position d'imagerie ;
des moyens de support isocentriques (5, 6, 9, 10 ; 116, 117, 118), reliés auxdits moyens d'imagerie isocentriques, pour supporter de manière mobile ledit instrument de ponction de sorte que ledit instrument de ponction est déplaçable sur une plage de positions de pointage comprenant une première position de pointage et une seconde position de pointage, où ledit instrument de ponction est pointé vers ledit isocentre dans chacune desdites première et seconde positions de pointage, où lesdits moyens d'imagerie isocentriques et lesdits moyens de support isocentriques partagent un isocentre commun ; et
des moyens de positionnement pour permettre un mouvement relatif entre ladite zone d'intérêt dans ledit sein du patient et ledit isocentre de sorte que ladite zone d'intérêt dans ledit sein du patient est située audit isocentre.

10. Appareil selon la revendication 9 où ledit moyen formant bras supporte ladite source de signaux d'imagerie, ledit récepteur de signaux d'imagerie et lesdits moyens formant support, et lesdits moyens de positionnement comprennent des moyens pour déplacer le moyen formant bras.

11. Appareil selon la revendication 9 ou l'une quelconque des revendications suivantes, comprenant en outre :
un moyen formant table (11) pour supporter ledit patient, où ledit moyen formant bras supporte ladite source de signaux d'imagerie et ledit récepteur de signaux d'imagerie de sorte que ladite source de signaux d'imagerie et ledit récepteur de signaux d'imagerie sont disposés du même côté dudit moyen formant table.

12. Appareil selon la revendication 9 ou l'une quelconque des revendications suivantes, comprenant en outre :
un moyen formant table (11) pour supporter ledit patient comprenant une ouverture par laquelle ledit sein du patient est amené à faire saillie de manière pendante, où ledit moyen formant bras supporte ladite source de signaux d'imagerie et ledit récepteur de signaux d'imagerie par rapport audit sein pendant de manière à permettre l'imagerie dudit sein pendant.

13. Appareil selon la revendication 9 ou l'une quelconque des revendications suivantes, où ledit moyen formant bras supporte ladite source de signaux d'imagerie et ledit récepteur de signaux d'imagerie pour un mouvement relatif entre eux, où une distance entre ladite source de signaux d'imagerie et ledit récepteur de signaux d'imagerie peut être modifiée en déplaçant (113) au moins un élément parmi ladite source de signaux d'imagerie et ledit récepteur de signaux d'imagerie.

14. Appareil selon la revendication 9 ou l'une quelconque des revendications suivantes où ledit appareil comprend en outre un moyen d'immobilisation (102) pour immobiliser ledit sein du patient.

15. Appareil selon la revendication 9 ou l'une quelconque des revendications suivantes où ledit système d'imagerie isocentrique comprend un moniteur associé avec ledit récepteur de signaux d'imagerie pour produire une imagerie sensiblement en temps réel.
